Europäisches Patentamt

European Patent Office

Office européen des brevets

(11) Numéro de publication : **0 420 972 B1**

# (12) FASCICULE DE BREVET EUROPEEN

(45) Date de publication du fascicule du brevet :
**30.06.93 Bulletin 93/26**

(51) Int. Cl.$^5$ : **C07C 315/00**

(21) Numéro de dépôt : **90907111.0**

(22) Date de dépôt : **05.04.90**

(86) Numéro de dépôt international :
**PCT/FR90/00241**

(87) Numéro de publication internationale :
**WO 90/12000 18.10.90 Gazette 90/24**

(54) **PROCEDE DE PREPARATION DE SULFONYLMETHANES ET DE LEURS DERIVES.**

(30) Priorité : **06.04.89 FR 8904503**

(43) Date de publication de la demande :
**10.04.91 Bulletin 91/15**

(45) Mention de la délivrance du brevet :
**30.06.93 Bulletin 93/26**

(84) Etats contractants désignés :
**AT BE CH DE DK ES FR GB IT LI LU NL SE**

(56) Documents cités :
**DE-A- 2 012 011
DE-A- 2 043 381
DE-A- 2 419 274
DE-A- 2 432 414
US-A- 3 776 960
US-A- 3 932 526
HOUBEN-WEYL, "Methoden der organischen
Chemie", édition 4, volume IX, 1955, Georg
Thieme Verlag, Stuttgart, DE, pages 255-257**

(73) Titulaire : **CENTRE NATIONAL DE LA
RECHERCHE SCIENTIFIQUE
15, quai Anatole France
F-75007 Paris (FR)**
Titulaire : **HYDRO-QUEBEC
75, Boulevard René Levesque Ouest
Montréal Québec H2Z 1A4 (CA)**

(72) Inventeur : **ARMAND, Michel
"Les Corjons"
F-38410 S.-Martin-d'Uriage (FR)**

(74) Mandataire : **Sueur, Yvette et al
Cabinet SUEUR & L'HELGOUALCH, 78, Rue
Carnot
F-95240 Cormeilles-en-Parisis (FR)**

EP 0 420 972 B1

## Description

La présente invention concerne un procédé de synthèse de sulfonylméthanes et de leurs dérivés, et plus particulièrement de perfluorosulfonylméthanes symétriques et de leurs dérivés.

Les perfluorosulfonylméthanes répondant à la formule générale $[(R_FSO_2)CH]_y$, dans laquelle M désigne un métal ou un groupement ammonium quaternaire ou non, les $R_F$ identiques dans le cas de méthanes symétriques ou différents dans le cas de méthanes dissymétriques, représentent des radicaux monovalents perfluorohydrocarbyles et notamment des radicaux perfluoroalcoyles tels que $CF_3$, $C_2F_5$, $C_4F_9$ ou des radicaux perfluoroaryles tels que $C_6F_5$, et y est un nombre égal à la valence de M, sont intéressants par les propriétés liées à l'anion correspondant. En effet, la délocalisation de la charge de l'anion sur plusieurs centres électronégatifs, à savoir les atomes F, O et C, induit une basicité et un caractère nucléophile faibles. La stabilité des liaisons covalentes permet de plus un domaine étendu de stabilité redox, en particulier aux potentiels anodiques. Les sels de métaux alcalins, et notamment de lithium, dérivés des perfluorosulfonylméthanes sont en particulier utilisables pour former des solutions solides avec des matériaux macromoléculaires du type des polyéthers, lesdites solutions solides trouvant une application comme électrolytes solides polymères dans la production de générateurs primaires ou secondaires tout-solide en films minces.

Ces composés $Mg[(R_FSO_2)_2CH]_y$ s'obtiennent directement à partir de $R_FSO_2F$ par action sur l'organomagnésien $CH_3MgCl$ dans le THF [R.J. Koshar & R.A. Mitsch J. Org. Chem. 38.3358 (1973), DE 2 012 011 et US 3 776 960].

Toutefois, les organo-magnésiens sont des composés d'emploi délicat, car ils sont sensibles à l'air. En outre, ils sont onéreux.

On a maintenant trouvé un nouveau procédé de synthèse de sulfonylméthanes symétriques à partir d'halogénures de sulfonyle et de carbures ioniques, produits d'usage courant et d'un emploi aisé. Ce procédé est d'une grande simplicité à toute échelle et met en oeuvre les carbures qui sont des composés de faible coût, d'emploi aisé (solides) et obtenus facilement par réaction directe des éléments (carbone + métal).

L'invention a pour objet un procédé de synthèse de sulfonylméthanes ou de leurs dérivés répondant à la formule :

$$M[(RSO_2)_2CH]_m \qquad (I),$$

dans laquelle M représente H, un métal alcalin, un métal alcalino-terreux, un ion ammonium quaternaire $NR'_4$, dans lequel les radicaux R', identiques ou différents, sont des radicaux organiques monovalents choisis parmi les radicaux aliphatiques ayant de 1 à 8 atomes de carbone, les radicaux aryliques ou alicycliques ayant de 3 à 8 atomes de carbone, R représente un radical organique monovalent choisi parmi les radicaux aliphatiques ayant de 1 à 8 atomes de carbone, les radicaux aryliques ou alicycliques ayant de 3 à 8 atomes de carbone et m représente la valence de M, caractérisé en ce que

- l'on fait réagir un carbure ionique choisi parmi le carbure d'aluminium, le carbure de béryllium, le carbure de thorium ou le carbure d'uranium, avec un halogénure de sulfonyle répondant à la formule générale $RSO_2X$ dans laquelle R a la signification donnée ci-dessus et X est Cl ou F, la réaction entre le carbure ionique et l'halogénure de sulfonyle étant effectuée dans un solvant aprotique polaire,
- l'on effectue l'hydrolyse du produit obtenu,
- l'on ajoute au milieu réactionnel, avant ou après l'hydrolyse, un composé répondant à la formule $M_yY_m$ dans laquelle M et m ont la signification ci-dessus, Y représente un anion capable de former, avec le cation du carbure ionique, un composé qui peut être séparé du composé (I), et y représente la valence de Y.

Le procédé est mis en oeuvre à une température comprise entre 0°C et 150°C. Généralement, une température inférieure à 40°C convient.

Les carbures ioniques appropriés pour le procédé de l'invention sont des composés qui contiennent l'entité $C^{4-}$ et qui s'hydrolysent facilement pour produire $CH_4$.

Parmi ces carbures, le carbure d'aluminium est particulièrement préféré, car il facilement accessible et non toxique.

La réaction du carbure d'aluminium avec un halogénure de sulfonyle donne un sulfonyle carbure d'aluminium qui est difficile à isoler du fait de la forte interaction des ions aluminium avec les solvants. Ce composé est toutefois avantageusement utilisé tel qu'obtenu en solution comme intermédiaire pour la synthèse des composés (I).

Lorsque le composé (I) à synthétiser est un dérivé de sulfonylméthane $M[(RSO_2)_2CH]_m$, l'on ajoute au milieu réactionnel un sel répondant à la formule $M_yY_m$, M étant différent de H. L'anion Y est choisi de telle sorte que le sel d'aluminium formé avec cet anion puisse être séparé du dérivé de sulfonylméthane final. Les carbonates, les fluorures et les phosphates sont particulièrement préférés car leur sel d'aluminium est insoluble dans l'eau et dans la plupart des solvants des sulfonylméthanes. Il peut donc être séparé par filtration.

Lorsque le composé (I) à synthétiser est un sulfonyle méthane $(RSO_2)_2CH_2$, le composé $M_yY_m$ est un acide $H_yY$. De préférence, $H_yY$ est un acide non volatil tel que l'acide sulfurique ou l'acide phosphorique.

Le procédé peut également être mis en oeuvre en utilisant pour $M_yY_m$ un chlorure. Toutefois, dans un tel cas, l'élimination du chlorure d'aluminium obtenu est plus difficile, car sa solubilité est très proche de celle des composés souhaités. Il faut alors transformer le chlorure d'aluminium en HCl et en un autre sel d'aluminium par addition d'un acide fort tel que $H_2SO_4$ ou $H_3PO_4$. Il se forme alors un sel d'aluminium qui précipite, et HCl peut être séparé du sulfonylméthane ou de son dérivé par distillation fractionnée, car leurs points d'ébullition respectifs sont très différents.

Dans l'halogénure de sulfonyle $RSO_2X$ utilisé, le radical R peut être un radical chloré ou fluoré. Les radicaux fluorés sont particulièrement intéressants en électrochimie, notamment pour les générateurs. Les radicaux R particulièrement intéressants sont des radicaux alkyles perfluorés ayant de 1 à 4 atomes de carbone.

Les solvants aprotiques polaires peuvent être choisis parmi les éthers tels que le tétrahydrofuranne (THF), le diméthoxyéthane (DME), les glymes ; les amides tels que le diméthylformamide (DMF), la N méthylpyrolidone (NMP), la tétraméthylurée (TMU), la diméthyléthylèneurée (DMU), la tétraéthylsulfonamide (TESA), le diméthylsulfoxyde (DMSO).

Lorsque l'halogénure de sulfonyle utilisé pour la réaction est un fluorure de sulfonyle, on utilise de préférence les éthers DME et THF qui forment des milieux suffisamment solvatants pour permettre une réaction rapide. Lorsque l'halogénure de sulfonyle utilisé est un chlorure, l'emploi de solvants très polaires du type amide, purs ou sous forme de mélanges avec des éthers, est nécessaire.

L'agent d'hydrolyse est de préférence choisi parmi l'eau, l'acide chlorhydrique, l'acide sulfurique ou l'acide phosphorique.

Lorsque le produit à synthétiser est un sulfonylméthane $(RSO_2)_2CH_2$, l'agent d'hydrolyse est avantageusement choisi parmi l'acide sulfurique et l'acide phosphorique. Il peut alors remplir également la fonction du composé $M_yY_m$.

Le procédé selon l'invention permet d'accéder simplement à de nombreux sels de sulfonylméthane à partir de carbures ioniques, et notamment de carbure d'aluminium.

La présente invention sera décrite plus en détail à l'aide des exemples suivants donnés à titre illustratif non limitatif.

EXEMPLE 1

Préparation du sel de potassium du bis(trifluorométhanesulfonyl)méthane

22 ml de chlorure de trifluorométhanesulfonyle ont été ajoutés à une suspension de 4,8 g de carbure d'aluminium et de 24 g de fluorure de potassium dans 200 ml d'un mélange de DMF et de DME (50/50). La réaction s'est produite sous agitation à température ordinaire en 72 heures. La suspension a été filtrée après ajout de 20 ml d'eau, puis évaporée sous vide.

Après extraction à l'acétone, on a obtenu 23 g du sel de potassium $K(CF_3SO_2)_2CH$ correspondant à un rendement de 72 %.

EXEMPLE 2

Préparation du sel de sodium du bis(trifluorométhanesulfonyl)méthane

Dans un autoclave contenant 48 g de carbure d'aluminium en poudre fine en suspension dans 450 ml de diglyme et maintenu à -20°C, on a introduit 304 g de fluorure de trifluorométhanesulfonyle. Le récipient a été fermé et agité à la température de 100°C jusqu'à l'observation d'une chute de la pression dans le réacteur. Après refroidissement et ouverture du récipient, on a ajouté 300 ml d'eau contenant 120 g de carbonate de sodium. La solution a été filtrée et évaporée pour obtenir 250 g du sel de sodium $Na(CF_3SO_2)_2CH$.

EXEMPLE 3

Préparation du bis(trifluorométhanesulfonyl)méthane

20 g du sel de sodium de l'exemple 2 ont 2 été traités par 5 cc d'acide sulfurique anhydre. Par distillation sous pression réduite ($130°C/1,3 \times 10^4 Pa$), on a obtenu 17 g de TSFM $(CF_3SO_2)_2CH_2$, correspondant à un rendement de 91 %.

EXEMPLE 4

Préparation directe du bis(perfluorobutane sulfonyl)méthane

Dans un autoclave contenant 24 g de carbure d'aluminium en suspension dans un mélange équivolumique de diglyme et de TMU, on a ajouté 302 g de fluorure de perfluorobutane sulfonyle. le récipient a été fermé et porté à 80°C pendant 72 h. Après refroidissement, on a ajouté 200 ml d'eau acidifiée par 50 ml d'acide sulfurique. On a ensuite extrait à l'éther 250 g d'un solide qui est le composé $(C_4F_9SO_2)_2CH_2$.

Les sels d'autres métaux peuvent être obtenus par action des métaux, oxydes, hydroxydes, carbonates correspondants.

Le procédé selon l'invention permet par conséquent de synthétiser directement, à partir de réactifs facilement accessibles et peu coûteux, de nombreux sulfonylméthanes ou leurs dérivés, en utilisant un carbure ionique.

A partir d'un dérivé obtenu directement, on peut préparer un sulfonyl méthane par réaction avec un acide.

A partir d'un sulfonyl méthane, obtenu directement ou indirectement, on peut obtenir un dérivé d'un autre cation par action de métaux, d'oxydes, d'hydroxydes ou de carbonates correspondants.

**Revendications**

1. Procédé de synthèse de sulfonylméthanes ou de leurs dérivés répondant à la formule

$$M[(RSO_2)_2CH]_m \qquad (I)$$

dans laquelle M représente H, un métal alcalin, un métal alcalino-terreux, un ion ammonium quaternaire $NR'_4$, dans lequel les radicaux R', identiques ou différents, sont des radicaux organiques monovalents choisis parmi les radicaux aliphatiques ayant de 1 à 8 atomes de carbone, les radicaux aryliques ou alicycliques ayant de 3 à 8 atomes de carbone, R représente un radical organique monovalent choisi parmi les radicaux aliphatiques ayant de 1 à 8 atomes de carbone, les radicaux aryliques ou alicycliques ayant de 3 à 8 atomes de carbone, et m représente la valence de M, caractérisé en ce que

- l'on fait réagir, dans un solvant polaire aprotique, un carbure ionique choisi parmi le carbure d'aluminium, le carbure de béryllium, le carbure de thorium ou le carbure d'uranium, avec un halogénure de sulfonyle répondant à la formule générale $RSO_2X$ dans laquelle R a la signification donnée ci-dessus et X est Cl ou F.
- l'on effectue l'hydrolyse du produit obtenu,
- l'on ajoute au milieu réactionnel, avant ou après l'hydrolyse, un composé répondant à la formule $M_yY_m$ dans laquelle M et m ont la signification donnée ci-dessus, Y représente un anion capable de former, avec le cation du carbure ionique, un composé qui peut être séparé du composé (I), et y représente la valence de Y.

2. Procédé selon la revendication 1, caractérisé en ce que le carbure ionique est le carbure d'aluminium.

3. Procédé selon l'une des revendications 1 ou 2, caractérisé en ce que le radical R est un radical chloré ou fluoré.

4. Procédé selon la revendication 3, caractérisé en ce que le radical R est un radical alkyle perfluoré ayant de 1 à 4 atomes de carbone.

5. Procédé selon l'une quelconque des revendications 1 à 4, caractérisé en ce que le composé $M_yY_m$ est un sel, M étant différent de H, le produit obtenu étant un sulfonyle carbure répondant à la formule $M[RSO_2)_2CH]_m$.

6. Procédé selon la revendication 5, caractérisé en ce que le sel $M_yY_m$ est choisi parmi les carbonates, les fluorures, les phosphates.

7. Procédé selon l'une quelconque des revendications 1 à 4, caractérisé en ce que le composé $M_yY_m$ est un acide non volatil $H_yY$, le composé obtenu étant un méthanesulfonyle répondant à la formule $(RSO_2)_2CH_2$.

8. Procédé selon la revendication 7, caractérisé en ce que $H_yY$ est choisi parmi l'acide sulfurique et l'acide phosphorique.

9. Procédé selon l'une quelconque des revendications 1 à 8, caractérisé en ce que le solvant polaire aprotique est choisi parmi les éthers, les amides, le diméthylsulfoxyde.

10. Procédé selon l'une quelconque des revendications 1 à 9, caractérisé en ce que l'agent d'hydrolyse est choisi parmi l'eau, l'acide chlorhydrique, l'acide sulfurique et l'acide phosphorique.

**Patentansprüche**

1. Verfahren zur Synthese von Sulfonylmethanen oder ihrer Derivate, die der Formel

$$M[(RSO_2)_2CH]_m \qquad (I)$$

entsprechen, in der M H, ein Alkalimetall, ein Erdalkalimetall, ein quarternäres Ammoniumion $NR'_4$, in dem die Reste R', die identisch oder verschieden sind, monovalente, organische Reste sind, die unter den aliphatischen Resten mit 1 bis 8 Kohlenstoffatomen, den arylischen oder alizyklischen Resten mit 3 bis 8 Kohlenstoffatomen ausgewählt sind, ist, R ein monovalenter, organischer Rest darstellt, der unter den aliphatischen Resten mit 1 bis 8 Kohlenstoffatomen, den arylischen oder alizyklischen Resten mit 3 bis 8 Kohlenstoffatomen ausgewählt ist, und m die Wertigkeit von M bedeutet, dadurch gekennzeichnet, daß

- man in einem polaren, aprotischen Lösungsmittel ein ionisches Karbid, das unter dem Aluminiumkarbid, dem Berylliumkarbid, dem Thoriumkarbid oder dem Urankarbid ausgewählt ist, mit einem Sulfonylhalogenid reagieren läßt, das der allgemeinen Formel $RSO_2X$ entspricht, in der R die oben gegebene Bedeutung besitzt und X Cl oder F ist,
- man eine Hydrolyse des erhaltenen Produkts durchführt,
- man dem Reaktionsmilieu vor oder nach der Hydrolyse eine Verbindung zufügt, die der Formel $M_yY_m$ entspricht, in der M und m die oben gegebene Bedeutung besitzen, Y ein Anion bedeutet, das in der Lage ist, mit dem Kation des ionischen Karbids eine Verbindung zu bilden, die von der Verbindung (I) getrennt werden kann, und y die Wertigkeit von Y bedeutet.

2. Verfahren nach Anspruch 1, dadurch gekennzeichnet, daß das ionische Karbid das Aluminiumkarbid ist.

3. Verfahren nach einem der Ansprüche 1 oder 2, dadurch gekennzeichnet, daß der Rest R ein chlorierter oder fluorierter Rest ist.

4. Verfahren nach Anspruch 3, dadurch gekennzeichnet, daß der Rest R ein perfluorierter Alkylrest mit 1 bis 4 Kohlenstoffatomen ist.

5. Verfahren nach einem der Ansprüche 1 bis 4, dadurch gekennzeichnet, daß die Verbindung $M_yY_m$ ein Salz ist, wobei M von H verschieden ist, wobei das erhaltene Produkt ein Sulfonylkarbid ist, daß der Formel $M[(RSO_2)_2)CH]_m$ entspricht.

6. Verfahren nach Anspruch 5, dadurch gekennzeichnet, daß das Salz $M_yY_m$ unter den Karbonaten, den Fluoriden und den Phosphaten ausgewählt wird.

7. Verfahren nach einem der Ansprüchen 1 bis 4, dadurch gekennzeichnet, daß die Verbindung $M_yY_m$ eine nicht flüchtige Säure $H_yY$ ist, wobei die erhalten Verbindung ein Methansulfonyl ist, das der Formel $(RSO_2)_2CH_2$ entspricht.

8. Verfahren nach Anspruch 7, dadurch gekennzeichnet, daß $H_yY$ unter der Schwefelsäure und der Phosphorsäure ausgewählt wird.

9. Verfahren nach einem der Ansprüche 1 bis 8, dadurch gekennzeichnet, daß das polare, aprotische Lösungsmittel unter den Äthern, den Amiden und dem Dimethylsulfoxyd ausgewählt wird.

10. Verfahren nach einem der Ansprüche 1 bis 9, dadurch gekennzeichnet, daß das Hydrolysemittel unter dem Wasser, der Hydrochlorsäure, der Schwefelsäure und der Phosphorsäure ausgewählt wird.

**Claims**

1. A process for the synthesis of sulfonylmethanes or derivatives thereof of the formula I :

$$M[(RSO_2)_2CH]_m \qquad (I)$$

wherein M represents H, an alkali metal, an alkaline earth metal, a quaternary ammonium ion $NR'_4$, wherein the radicals R', identical or different, are monovalent organic radicals selected from aliphatic radicals having from 1 to 8 carbon atoms, arylic or alicyclic radicals having from 3 to 8 carbon atoms, R represents a monovalent organic radical selected from aliphatic radicals having from 1 to 8 carbon atoms, arylic or alicyclic radicals having from 3 to 8 carbon atoms, et m represents the valence of M, characterized in that :

- an ionic carbid selected from aluminium carbide, beryllium carbide, thorium carbide or uranium carbide, is reacted, in a polar aprotic solvent, with a sulfonyl halide of the general formula $RSO_2X$ wherein R is as defined above and X is Cl or F,
- the product obtained is hydrolysed,
- a compound of the formula $M_yY_m$ is added to the reaction mixture, before or after hydrolysis, wherein M and m are as defined above, Y represents an anion capable of forming with the cation of the ionic carbide a compound which can be separated from compound (I) and wherein y represents the valence of Y.

2. The process according to claim 1, characterized in that the ionic carbide is aluminium carbide.

3. The process according to claim 1 or claim 2, characterized in that radical R is a chlorinated or a fluorinated radical.

4. The process according to claim 3, characterized in that radical R is a perfluorinated alkyl radical having from 1 to 4 carbon atoms.

5. The process according to any of claims 1 to 4, characterized in that the compound $M_yY_m$ is a salt, M being different from H, the product obtained being a sulfonyl carbide of the formula $M[(RSO_2)_2CH]_m$.

6. The process according to claim 5, characterized in that the salt $M_yY_m$ is selected from carbonates, fluorides, phosphates.

7. The process according to any of claims 1 to 4, characterized in that the compound $M_yY_m$ is a non volatil acid $H_yY$, the product obtained being a methanesulfonyl of the formula $(RSO_2)_2CH_2$.

8. The process according to claim 7, characterized in that $H_yY$ is selected from sulfuric acid and phosphoric acid.

9. The process according to any of claims 1 to 8, characterized in that the polar aprotic solvent is selected from ethers, amides, dimethylsulfoxide.

10. The process according to any of claims 1 to 9, characterized in that the hydrolyzing agent is selected from water, hydrochloric acid, sulfuric acid and phosphoric acid.